# EUROPEAN PATENT APPLICATION

(11) **EP 1 350 779 A1**
(43) Date of publication of application: **08.10.2003**
(21) Application number: 03075755.3
(22) Date of filing: 17.03.2003
(51) Int. Cl.: C07C 6/02, B01J 23/36, B01J 37/08

(54) **Preparation of a supported catalyst based on rhenium and its use in the metathesis reaction of olefins**

(30) Priority: 20.03.2002 IT MI20020582
(71) Applicant: Polimeri Europa S.p.A., 72100 Brindisi (IT)
(72) Inventor: Querci, Cecilia, 28100 Novara (IT); Panella, Francesco, 13030 Pertengo (Vercelli) (IT); Guerrini, Rinaldo, 28100 Novara (IT); Russo, Matteo, 28065 Cerano (Novara) (IT)
(74) Representative: De Gregori, Antonella

(57) **Abstract**

A process is described for the preparation of a heterogeneous catalyst containing rhenium as active component and an inert carrier, characterized in that said inert carrier is previously subjected to thermal treatment before the supporting of the active component and the activation of the heterogeneous catalyst is effected by means of thermal treatment followed by a rapid final cooling.
The catalyst is particularly active in the metathesis reaction of olefins.

## Description

The present invention relates to a process for the preparation of a heterogeneous catalyst containing rhenium as active component and an inert carrier, characterized in that said inert carrier is previously subjected to thermal treatment, before the supporting of the active component, and the activation of the heterogeneous catalyst is effected by means of thermal treatment followed by a rapid final cooling.

The present invention also relates to the use of said catalyst in the metathesis reaction of olefins.

The metathesis reaction, also known as dismutation and disproportionation of olefins, is a reaction of great practical interest which can be used, for example, for balancing the weight of olefins resulting from steam cracking.

When olefins are treated in the presence of suitable catalysts, they are converted to other olefins in a reaction in which the alkylidene groups (R¹R²C=) are inter-exchanged with a process schematically represented by the following equation:

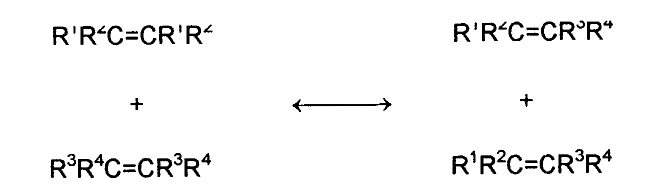

Heterogeneous catalysts essentially consisting of rhenium derivatives supported on inert materials (for example silica or alumina) are known to be active in the metathesis of olefins. For example, US 3,641,189 and US 3,676,520 describe the preparation of these materials and their use in the metathesis of olefins.

In the preparation of this catalyst, the active component is normally introduced onto the surface of the carrier through impregnation. In this reaction, the carrier is mixed with a solution in which the active component has been dissolved. The active component remains inside the carrier particles when the solvent is removed by evaporation. The use of a quantity of solution equal to the pore volume, prevents the compound from crystallizing in the space between the particles.

With these catalysts, however, not particularly high yields have been observed and, in the case of higher olefins, there are also poor selectivities, often due to secondary isomerization reactions of double bonds (J. Mol. Cat: 46, 1988, 119-130 and App. Catal., 70, 1991, 295-306).

It has now been found that it is possible to overcome the above-mentioned drawbacks by means of the catalyst of the present invention containing rhenium as active component and an inert carrier, characterized in that said carrier is subjected to a previous thermal treatment before its impregnation with the active component and the activation of the heterogeneous catalyst is effected by thermal treatment followed by a rapid final cooling.

This catalyst is active in metathesis reactions even when used in the absence of a co-catalyst and allows problems due to the formation of isomers or side reactions to be reduced, giving a high selectivity.

In accordance with the above, an objective of the present invention relates to a process for the preparation of a heterogeneous catalyst active in the metathesis reaction of olefins, containing rhenium as active component and an inert carrier, characterized in that the inert carrier is subjected to a thermal treatment at a temperature ranging from 100 to 600°C, in flowing air, before being supported with the active component, and the activation of the heterogeneous catalyst is effected by thermal treatment followed by a rapid final cooling.

The thermal treatment of the carrier is preferably carried out in two subsequent steps, a pre-calcination step at a temperature ranging from 100 to 200°C in flowing air, for a time ranging from 30 minutes to 2 hours and a subsequent calcination at a temperature ranging from 300 to 600°C in flowing air, for a time ranging from 1 to 6 hours.

The carrier used for the catalyst according to the present invention can be selected from the group consisting of refractory oxides and/or aluminosilicalite which can be of basic, acid or neutral nature.

Examples of said carriers can be alumina, silica, silica-alumina. Alumina is preferably used with a surface area ≥ 50 m²/g, preferably from 100 to 200 m²/g, and total cumulative pore volume higher than 0.1 ml/g, preferably from 0.3 to 0.8 ml/g.

The rhenium compound can be introduced into the pre-treated carrier as mentioned above, by means of precipitation or impregnation starting from precursors consisting, for example, of solutions of its salts or soluble complexes.

The rhenium compound precursors are selected from rhenium heptoxide, ammonium perrhenate, tetra alkyl ammonium perrhenate, perrhenic acid, etc.

The impregnation of the inert carrier using a saturated solution of the rhenium compound, in a solvent selected from water or an organic solvent, for example a hydrocarbon, an alcohol or an ether, is generally preferred.

The impregnation is preferably carried out at a temperature of between 20 and 70°C in order to increase the solubility of the rhenium salt; in this case, the carrier is also heated to the same temperature.

After the impregnation of the carrier with the metal, the catalyst is activated by a pre-calcination at a temperature ranging from 100 to 200°C under a flow of dry air and a subsequent calcination at a temperature of between 300 and 600°C, under a flow, first of dry air and subsequently of nitrogen. The cooling is carried out in flowing nitrogen for a time ranging from 5 to 30 minutes, preferably between 10 and 20 minutes.

Rhenium is normally present in these catalysts in a quantity of between 1 and 20% by weight, preferably between 3 and 10% by weight with respect to the carrier.

The catalysts of the present invention can be used in metathesis reactions of olefins.

Said reactions can be homo-metathesis (when the two olefins are the same) or co-metathesis (when the two olefins are different).

The olefins which can be subjected to metathesis reactions are mono-olefins having from 2 to 30 carbon atoms, such as, for example, ethylene, propylene, butene, pentene, hexene; cyclo-olefins having from 3 to 20 carbon atoms, for example cyclopentene, cyclo-octene, norbornene; polyolefins having from 4 to 30 carbon atoms, for example 1,4-hexadiene, 1,7-octadiene, cyclo-polyolefins having from 5 to 30 carbon atoms, for example 1,5-cyclo-octadiene, norbardiene, dicyclopentadiene.

Other olefins are mono- or poly-olefins, linear or cyclic, carrying functional groups, such as, for example, halogens or ester groups such as methyl oleate.

The metathesis reaction can be carried out both in batch and in continuous operations, by feeding the materials into a fluid bed or fixed bed reactor. The reaction conditions, such as temperature, pressure and flows are selected in relation to the material fed and the end-product to be obtained.

The metathesis reaction is normally carried out at a temperature ranging from 0 to 100°C, preferably from 25 to 60°C, and a pressure of 0 to 100 bar, preferably from 1 to 60 bar and can be carried out in gas or liquid phase, with or without an organic solvent.

When a solvent is used, this is selected from ethers, aliphatic and aromatic hydrocarbons. Examples of these solvent are: ethyl ether, hexane, heptane, toluene, etc.

The catalyst is normally dispersed in the reaction medium at a concentration ranging from 1 to 50% by weight, preferably from 1 to 10% by weight, with respect to the reaction mixture.

The metathesis reaction can be optionally carried out in the presence of co-catalysts selected from alkyl metals such as, for example, tin tetra-alkyls (tin tetra-methyl, tin tetra-ethyl, tin tetra-butyl), or other alkyl metals such as lead tetra-methyl, lead tetra-ethyl, aluminum triethyl, chloro-aluminum diethyl, as described in US patent 3,855,338.

The following examples are illustrative but non-limiting of the invention.

In the examples, the conversions (well beyond the equilibrium concentration) were measured while removing the undissolved ethylene which is produced with 5-decene in 1-hexene metathesis.

### Example 1

### Preparation of the catalyst A

10 g of γ alumina with a specific surface of 180 m²/g and a porosity of 0.5 ml/g, are pre-calcined in a muffle at 110°C for 1 hour in flowing air and subsequently at 550°C for 4 hours in flowing air.

The carrier is then wetted four times with 5 ml of a water solution containing 0.28 g of NH₄ReO₄, and between one impregnation and another, the water is evaporated maintaining the sample in an oven at 60°C.

The catalyst is calcined first at 110°C for 1 hour in a flow of dry air and subsequently at 550°C for 3 hours in a flow of dry air and 1 hour in flowing nitrogen. The reactor is then extracted from the muffle and is cooled for 15 minutes in flowing nitrogen.

The catalyst thus prepared has a rhenium content of 7.5% by weight.

### Example 2

### Use of catalyst A in metathesis

358 mg of catalyst A prepared as in example 1 and 23 ml of a solution consisting of 10 µl of co-catalyst SnMe₄ in 100 ml of hexane are charged into a 150 ml tailed flask, in an argon atmosphere.

The resulting mixture is maintained under light stirring, at 25°C for 10 minutes and 26 ml of 1-hexene are subsequently added.

The reaction mixture is analyzed, after 30 minutes, by means of gas chromatography, using an internal standard. The following results are obtained:
- conversion of 1-hexene 70%
- selectivity to 5-decene 100%

### Example 3

### Use of catalyst A in the absence of a co-catalyst

358 mg of catalyst A and, subsequently, 23 ml of hexane and 26 ml of 1-hexene are charged into a 150 ml tailed flask, in an argon atmosphere.

The mixture is maintained under stirring, at 25°C for 30 minutes and then analyzed by means of gas chromatography, using an internal standard.

The conversion of 1-hexene is 20% with a selectivity to 5-decene of 100%.

### Example 4 (comparison)

### Preparation of catalyst B

10 γ of alumina with a specific surface of 180 m²/g and a porosity of 0.5 ml/g, are used without previous calcination.

The carrier is then wetted four times with 5 ml of a water solution containing 0.28 g of NH₄ReO₄, and between one impregnation and another, the water is evaporated by maintaining the sample in an oven at 60°C.

The catalyst is calcined first at 110°C for 1 hour in a flow of dry air and subsequently at 550°C for 3 hours in a flow of dry air and 1 hour in flowing nitrogen. The reactor is extracted from the muffle and is cooled for 15 minutes in flowing nitrogen.

The catalyst thus prepared has a rhenium content of 7.5% by weight.

### Example 5 (comparison)

### Use of catalyst B in metathesis

358 mg of catalyst B prepared as in example 4 and 23 ml of a solution consisting of 10 µl of co-catalyst SnMe₄ in 100 ml of hexane are charged into a 150 ml tailed flask, in an argon atmosphere.

The resulting mixture is maintained under light stirring, at 25°C for 10 minutes and 26 ml of 1-hexene are subsequently added.

The reaction mixture is analyzed, after 30 minutes, by means of gas chromatography and no transformation of hexene is observed.

### Example 6 (comparison)

### Preparation of catalyst C

The catalyst is prepared operating as in example 1, but using a calcined γ alumina in an air flow at 250°C before impregnation.

### Example 7 (comparison)

### Use of catalyst C in metathesis

358 mg of catalyst C prepared as in example 6 and 23 ml of a solution consisting of 10 µl of co-catalyst SnMe₄ in 100 ml of hexane, are charged into a 150 ml tailed flask, in an argon atmosphere.

The resulting mixture is maintained under light stirring, at 25°C for 10 minutes and 26 ml of 1-hexene are subsequently added.

The reaction mixture is analyzed, after 30 minutes, by means of gas chromatography, using an internal standard. The following results are obtained:
- conversion of 1-hexene 3%
- selectivity to 5-decene 100%.

### Example 8 (comparison)

### Preparation of catalyst D

The same procedure is adopted as in example 1, using a nitrogen flow instead of air in the precalcination and calcination of the carrier.

10 γ of alumina with a specific surface of 180 m²/g and a porosity of 0.5 ml/g, are calcined in a muffle at 110°C for 1 hour in flowing nitrogen and subsequently at 550°C for 4 hours again in flowing nitrogen.

The carrier is then wetted four times with 5 ml of a water solution containing 0.28 g of NH₄ReO₄, and between one impregnation and another, the water is evaporated by maintaining the sample in an oven at 60°C.

The catalyst is calcined first at 110°C for 1 hour in a flow of dry air and subsequently at 550°C for 3 hours in a flow of dry air and 1 hour in flowing nitrogen. The reactor is extracted from the muffle and is cooled for 15 minutes in flowing nitrogen.

The catalyst thus prepared has a rhenium content of 7.5% by weight.

### Example 9 (comparison)

### Use of catalyst D in metathesis

358 mg of catalyst D prepared as in example 8 and 2.3 ml of a solution consisting of 10 µl of co-catalyst SnMe₄ in 10 ml of hexane are charged into a 150 ml tailed flask, in an argon atmosphere.

The resulting mixture is maintained under light stirring, at 25°C for 10 minutes and 2.6 ml of 1-hexene are subsequently added.

The reaction mixture is analyzed, after 30 minutes, by means of gas chromatography, using an internal standard. No transformation of hexene is observed.

### Example 10

### Preparation of catalyst E

The catalyst is prepared operating as in example 1, but the reactor is maintained in the muffle and cooled down over a period of 5 hours in flowing nitrogen.

The catalyst thus prepared has a rhenium content of 7.5% by weight.

### Example 11 (comparison)

### Use of catalyst E in metathesis

358 mg of catalyst E prepared as in example 10 and 23 ml of a solution consisting of 10 µl of co-catalyst SnMe₄ in 100 ml of hexane are charged into a 150 ml tailed flask, in an argon atmosphere.

The resulting mixture is maintained under light stirring, at 25°C for 10 minutes and 26 ml of 1-hexene are subsequently added.

The reaction mixture is analyzed, after 30 minutes, by means of gas chromatography, using an internal standard. The following results are obtained:
- conversion of 1-hexene 7%
- selectivity to 5-decene 100%.
Table 1 shows the results obtained in the examples.

**Table 1**

| Example | Catalyst | Co-catalyst | Hexene % conversion | 5-decene selectivity |
|---|---|---|---|---|
| 2 | A | SnMe₄ | 70 | 100 |
| 3 | A | 0 | 20 | 100 |
| 5 | B | SnMe₄ | 0 | 0 |
| 7 | C | SnMe₄ | 3 | 100 |
| 9 | D | SnMe₄ | 0 | 0 |
| 11 | E | SnMe₄ | 7 | 100 |

From the values shown in the table, it can be observed that catalyst A shows a high activity and selectivity both in the presence and in the absence of the co-catalyst (examples 2 and 3).

From the table it can also be noted that the catalyst does not show any activity (examples 5 and 9) or a very low activity (examples 7 and 11), when the supporting or the cooling times of the heterogeneous catalyst are effected under different conditions with respect to those of the present invention.

## Claims

1. A process for the preparation of a heterogeneous catalyst active in the metathesis reaction of olefins, containing rhenium as active component and an inert carrier, **characterized in that** the inert carrier is subjected to a thermal treatment at a temperature ranging from 100 to 600°C, in flowing air, before being supported with the active component, and the activation of the heterogeneous catalyst is effected by thermal treatment followed by a final cooling carried out over a time ranging from 5 to 30 minutes.

2. The process according to claim 1, wherein the thermal treatment of the inert carrier is carried out by means of two subsequent steps:
(i) a first step wherein the carrier is subjected to pre-calcination at a temperature ranging from 100 to 200°C in flowing air, over a time ranging from 30 minutes to 2 hours; and
(ii) a second step wherein the pre-calcined carrier is subjected to a temperature ranging from 300 to 600°C in flowing air, over a time ranging from 1 to 6 hours.

3. The process according to claim 1, wherein the carrier is selected from the group consisting of refractory oxides and/or aluminosilicalite which can be of a base, acid or neutral nature.

4. The process according to claim 3, wherein the carrier is selected from alumina, silica and silica-alumina.

5. The process according to claim 4, wherein the carrier is alumina.

6. The process according to claim 5, wherein the alumina has a specific surface area of ≥ 50 m²/g and a total cumulative pore volume > 0.01 ml/g.

7. The process according to claim 6, wherein the alumina has a specific surface area of between 100 and 200 m²/g and a total cumulative pore volume of between 0.3 and 0.8 ml/g.

8. The process according to claim 1, wherein the cooling of the heterogeneous catalyst is carried out at a temperature ranging from 10 to 20 minutes.

9. The process according to claim 1, wherein the active component rhenium is introduced onto the pre-treated carrier as specified in claims 1-7, through precipitation or impregnation starting from its precursors in the form of solutions of its salts or soluble complexes.

10. The process according to claim 9, wherein the rhenium precursors are selected from rhenium heptoxide, ammonium perrhenate, tetra alkyl ammonium perrhenate and perrhenic acid.

11. The process according to claim 1, wherein the catalyst contains a quantity of rhenium ranging from 1 to 20% by weight with respect to the carrier.

12. The process according to claim 11, wherein the catalyst contains a quantity of rhenium ranging from 3 to 10% by weight.

13. The process according to claim 1, wherein the supported catalyst containing rhenium is activated through a pre-calcination at a temperature ranging from 100 to 200°C in flowing air and a subsequent calcination at a temperature ranging from 300 to 600°C in a flow of dry air and subsequently nitrogen.

14. A process for the conversion of olefins by means of a metathesis reaction, **characterized in that** it is carried out in the presence of a catalyst according to claim 1.

15. The process according to claim 14, wherein the metathesis reaction can be homo-metathesis or co-metathesis.

16. The process according to claim 14, wherein the olefins are selected from mono-olefins having from 2 to 30 carbon atoms, cyclo-olefins having from 3 to 20 carbon atoms, polyolefins having from 4 to 30 carbon atoms, cyclo-polyolefins having from 5 to 30 carbon atoms.

17. The process according to claim 16, wherein the mono-olefins are selected from ethylene, propylene, butene, pentene and hexene.

18. The process according to claim 16, wherein the cyclo-olefins are selected from cyclopentene, cyclo-octene, norbornene.

19. The process according to claim 16, wherein the polyolefins are selected from 1,4-hexadiene and 1,7-octadiene.

20. The process according to claim 16, wherein the cyclo-polyolefins are selected from 1,5-cyclo-octadiene, norbardiene, dicyclo-pentadiene.

21. The process according to claim 16, wherein the linear or cyclic mono-olefins or polyolefins can carry functional groups such as, for example, halogens or ester groups such as methyl oleate.

22. The process according to claim 14, wherein the metathesis reaction is carried out at a temperature ranging from 0 to 100°C and a pressure of between 0 and 100 bar.

23. The process according to claim 22, wherein the metathesis reaction is carried out at a temperature ranging from 25 to 60°C and a pressure of between 1 and 60 bar.

24. The process according to claim 14, wherein the metathesis reaction is carried out in gas phase or in liquid phase, with or without a solvent selected from ethers, aliphatic and aromatic hydrocarbons.

25. The process according to claim 24, wherein the solvent is selected from ethyl ether, hexane, heptane, toluene.

26. The process according to claim 14, wherein the quantity of catalyst ranges from 1 to 50% by weight with respect to the reaction mixture.

27. The process according to claim 26, wherein the quantity of catalyst ranges from 1 to 10% by weight with respect to the reaction mixture.

28. The process according to claim 14, wherein the metathesis reaction is carried out batchwise or in continuous.
